(19) Europäisches Patentamt

**European Patent Office**

**Office européen des brevets**

(11) Numéro de publication: **0 249 532 B1**

(12) **FASCICULE DE BREVET EUROPEEN**

(45) Date de publication de fascicule du brevet: **02.09.92**  (51) Int. Cl.⁵: **A61N 1/06**

(21) Numéro de dépôt: **87401238.8**

(22) Date de dépôt: **03.06.87**

(54) **Dispositif pour l'application de courants haute fréquence.**

(30) Priorité: **11.06.86 FR 8608535**

(43) Date de publication de la demande:
**16.12.87 Bulletin 87/51**

(45) Mention de la délivrance du brevet:
**02.09.92 Bulletin 92/36**

(84) Etats contractants désignés:
**AT BE CH DE ES GB GR IT LI LU NL SE**

(56) Documents cités:
**WO-A-80/01045**
**DE-C- 655 302**
**FR-A- 741 062**

(73) Titulaire: **S.A.E.A.T. - FLUVITA**
**32, rue Stalingrad**
**Le Pré Saint Gervais La Seine Saint**
**Denis(FR)**

(72) Inventeur: **Ory, Michel Paul**
**32 rue de Stalingrad**
**Le Pré Saint Gervais La Seine St. Denis(FR)**

(74) Mandataire: **Cabinet Pierre HERRBURGER**
**115, Boulevard Haussmann**
**F-75008 Paris(FR)**

Rank Xerox (UK) Business Services

## Description

La présente invention a pour objet un dispositif pour l'application de courants haute fréquence sans manipulation dans des soins médicaux, esthétiques et en kinésithérapie.

Ce dispositif se compose d'une manière usuelle d'au moins une électrode en une matière isolante rigide à l'intérieur de laquelle règne un vide plus ou moins poussé qui est reliée à un câble conducteur.

Les praticiens utilisent depuis déjà de nombreuses années, les électrodes dites de "MAC INTIRE" qui sont constituées par des tubes en verre ou en pyrex (marque déposée) contenant un vide plus ou moins poussé. Lors de la mise en oeuvre de ces électrodes, on applique la propriété bien connue des gaz qui sont d'excellents isolants à la pression atmosphérique mais deviennent conducteurs lorsqu'ils se raréfient : lorsque ces électrodes sont alimentées par des courants dont la fréquence varie approximativement entre 50 et 300 kHz, il se crée un courant qui "s'enroule" autour du tube isolant ; ce phénomène est bien connu sous le nom de "skin effect". Plus la dépression est importante, plus l'électrode est conductrice.

Pour utiliser les courants ainsi créés dans le domaine des soins médicaux, esthétiques et en kinésithérapie, l'opérateur introduit l'électrode dans un manche spécial qu'il tient en main durant toute la durée du soin. Il s'agit là d'un processus très long et peu commode, étant donné qu'il exige un déplacement de l'électrode point par point avec quelques minutes d'application au même point. Il est facile de concevoir que, pour obtenir l'effet recherché, le traitement peut être très long, par exemple dans le cas d'un épaule ou d'une hanche. Cette caractéristique fait que les électrodes dites de "MAC INTIRE" sont pratiquement inutilisables pour le traitement de grandes surfaces ; ceci est d'autant plus vrai que, étant donné la durée nécessaire du traitement en chaque point, il peut apparaître des lésions ou brûlures de la peau par suite de courants trops intenses.

Pour remédier à cet inconvénient, on a proposé de "casser le vide" en introduisant dans les électrodes un gaz souvent coloré (argon, néon, fréon) de manière à pouvoir apprécier visuellement l'intensité de l'électrode. On n'a, toutefois, pas pu obtenir ainsi de résultats satisfaisants.

Un autre inconvénient des électrodes dites de "MAC INTIRE" est lié au fait qu'elles sont directement en contact avec la peau du patient et entraînent, par suite, un dégagement d'ozone particulièrement désagréable.

Par ailleurs, on a constaté que les courants qui se créent par "skin effect" à la surface des électro- des dites de "MAC INTIRE" sont susceptibles de faire pénétrer dans la peau du patient, un certain nombre de molécules même de grande dimension sans modification de leur structure atomique ; il s'agit là d'un domaine d'application important des électrodes dites de "MAC INTIRE" (procédé de cataphorèse - variante de l'électro-osmose) mais qui n'a pas jusqu'à présent connu le développement auquel on pouvait s'attendre, étant donné que, par ses propriétés oxydantes, l'ozone dégagé au niveau de la peau du patient risque de détériorer les principes actifs que l'on cherche à faire pénétrer à l'intérieur de la peau, détruisant parallèlement leurs propriétés.

Pour remédier à ces inconvénients, on a cherché à mettre au point des électrodes susceptibles de diminuer les temps de traitement en permettant une application non pas point par point, mais sur une partie de la surface du corps à traiter.

En tant qu'exemple de document décrivant un dispositif du type "MAC INTIRE", on peut citer le document FR-A-741 062 ; le dispositif décrit dans ce document est constitué par l'association d'électrodes en verre remplies de gaz et réunies en un seul ensemble par un support commun ; ces électrodes sont mises en place dans des saillies en forme de calottes prévues à cet effet dans le support. Par suite, ces électrodes se trouvent en contact direct avec la peau du patient à traiter. De plus, elles doivent obligatoirement, par suite de leur forme et de leur rigidité, être placées à une certaine distance les unes des autres, ce qui fait que leur nombre ne peut pas être augmenté à volonté.

La présente invention a pour objet de remédier à ces inconvénients en proposant un dispositif pour l'application de courants de fréquence environ comprise entre 50 et 300 kHz du type ci-dessus, caractérisé en ce qu'il comporte plusieurs électrodes constituées par des tubes en verre, placées côte à côte, dont le nombre et les dimensions correspondent à la partie du corps que l'on désire traiter, ces électrodes étant reliées par un élément conducteur souple, solidaire d'une douille conçue pour recevoir le câble conducteur, et l'ensemble ainsi obtenu étant noyé dans une matière isolante souple telle qu'un silicone susceptible de s'incurver pour s'adapter à la partie du corps à traiter, l'épaisseur de la couche de matière isolante placée entre les électrodes et la partie du corps à traiter, ou semelle, étant environ comprise entre 0,50 et 3,00 mm, de préférence de l'ordre de 2,00 mm.

Ce dispositif présente, vis-à-vis de l'art antérieur, deux avantages essentiels. Le premier de ces avantages est directement lié au fait que, étant donné qu'il est possible de placer les électrodes directement côte à côte, le dispositif est actif par toute sa surface et peut donc permettre de traiter très rapidement des zones importantes (épaule, hanche, cuisse). Le second avantage est directement lié à la présence entre les électrodes et la peau du patient d'une "semelle" en matière isolante qui empêche l'apparition de tout étincelage désagréable, et la production d'ozone.

Bien entendu, la forme et les dimensions du dispositif peuvent être quelconques et sont, avant tout, fonction de la surface à traiter ; on a toutefois utilisé de façon particulièrement avantageuse, de simples tubes en verre ou en pyrex (marque déposée) disposés côte à côte ; dans certains cas particuliers dans lesquels on cherche à obtenir une souplesse plus importante, ces tubes peuvent être remplacés par des billes.

Il est particulièrement avantageux, conformément à l'invention, de choisir un silicone en tant que matière isolante souple, compte tenu des propriétés isolantes et anallergiques de ce matériau et de l'absence de tout risque de réaction secondaire.

L'épaisseur de la couche de matière isolante placée entre les électrodes et la partie du corps à traiter ou semelle constitue, elle aussi, une caractéristique importante de l'invention ; elle doit en effet être choisie suffisante pour ne pas provoquer d'étincelage ou de dégagement d'ozone, mais parallèlement suffisamment faible pour ne pas nuire à l'effet thérapeutique recherché.

Selon une autre caractéristique de l'invention, l'élément conducteur est directement posé sur les électrodes. Celui-ci peut être constitué soit par un fil unique, soit le plus souvent par une tresse mise en place sur ou encore autour des électrodes.

Selon une autre caractéristique de l'invention, le dispositif comporte des sangles susceptibles de maintenir les électrodes et la matière isolante dans la position souhaitée en cours de traitement.

Selon l'invention, le dispositif susmentionné peut être fabriqué par un procédé par lequel on coule dans un moule une épaisseur de matière isolante correspondant à celle de la semelle, on place les électrodes sur celle-ci, puis l'élément conducteur souple et la douille, puis on coule la partie restante de la matière isolante de manière telle que les électrodes, l'élément conducteur et la partie inférieure de la douille se trouvent noyés dans cette matière et l'on démoule l'ensemble ainsi obtenu.

Il est, par suite, clair que le dispositif conforme à l'invention peut être fabriqué très facilement et pour un prix de revient peu élevé.

Les caractéristiques du dispositif qui fait l'objet de l'invention sont décrites plus en détail en se référant aux dessins annexés dans lesquels :

- la figure 1 représente, en coupe, le dispositif suivant l'invention,
- la figure 2 représente, en coupe, le dispositif en position incurvée,

- les figures 3a à 3c représentent les différentes étapes de mise en oeuvre du processus de fabrication du dispositif conforme à l'invention.

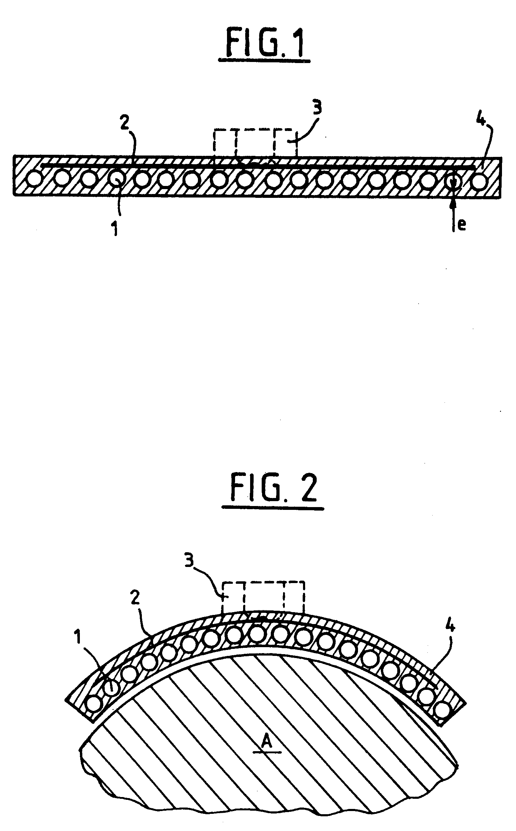

Selon la figure 1, le dispositif conforme à l'invention se compose de tubes en verre 1 notamment de tubes en pyrex (marque déposée) de longueur et de diamètre déterminés dans lesquels on a préalablement fait le vide. Ces tubes 1 sont placés côte à côte et reliés, les uns aux autres, par un élément conducteur, notamment une tresse conductrice 2 qui est simplement posée sur les tubes 1. Cette tresse est, de manière connue en elle-même, reliée à une douille 3 qui est spécialement conçue pour recevoir le câble conducteur non représenté sur les figures.

L'ensemble ainsi constitué est noyé dans une matière isolante souple 4, notamment un silicone ; l'épaisseur e de matière placée entre les électrodes 1 et la partie A du corps à traiter représentée en hachurés sur la figure 2, est de l'ordre de 2,00 mm. Cette épaisseur e est choisie de manière à obtenir un effet satisfaisant sans provoquer d'étincelage et de dégagement d'ozone désagréable.

Comme représenté sur la figure 2, le dispositif susmentionné peut être déformé pour épouser le corps A à traiter. Il est alors, de préférence, tenu en place à l'aide de sangles non représentées.

Les dimensions de ce dispositif peuvent, bien entendu, notablement varier en fonction des besoins.

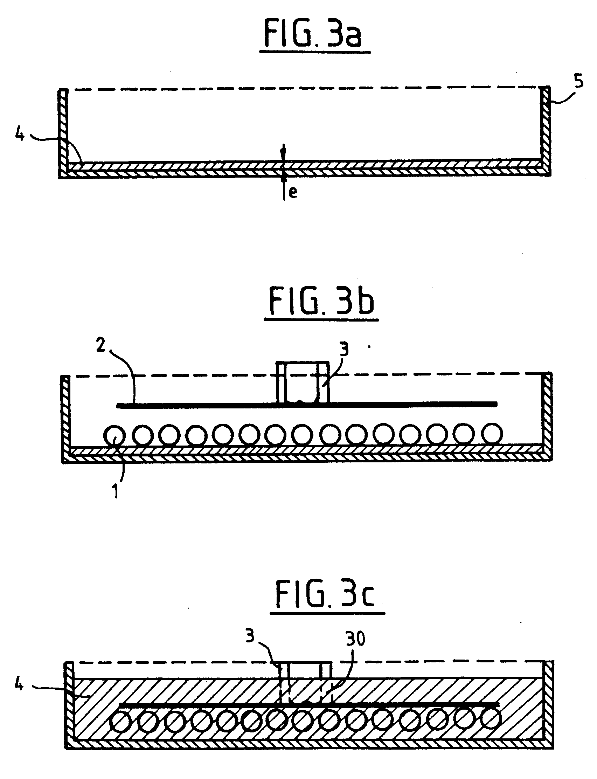

Selon la figure 3a, pour fabriquer le dispositif représenté sur les figures 1 et 2, on coule, dans un moule 5, dont les dimensions correspondent à celles du dispositif, une épaisseur e de matière isolante 4.

Selon la figure 3b, dans une seconde étape, on place, côte a cote, sur cette épaisseur e, les électrodes 1 que l'on surmonte par l'élément conducteur 2 que l'on relie à la douille 3.

Ensuite, selon la figure 3c, il ne reste plus qu'à couler la partie restante de la matière isolante 4 de manière telle que les électrodes 1, l'élément conducteur 2 et la partie inférieure 30 du la douille 3 se trouvent noyées dans la matière 4. On démoule ensuite l'ensemble ainsi obtenu qui est alors prêt à être utilisé.

## Revendications

1. Dispositif pour l'application de courants de fréquence environ comprise entre 50 et 300 kHz, sans manipulation, dans des soins médicaux, esthétiques et en kinésithérapie, se composant de plusieurs électrodes (1) constituées par des tubes en verre placés côte à côte à l'intérieur des quelles règne un vide plus ou moins poussé, ces électrodes (1) dont le nombre et les dimensions correspondent à la partie du corps (A) que l'on désire traiter, étant reliées par un élément conducteur souple (2) solidaire d'une douille (3) conçue pour recevoir le câble conducteur et l'ensemble ainsi obtenu étant noyé dans une matière isolante souple (4) telle qu'un silicone susceptible de s'incurver pour s'adapter à la partie du corps à traiter (A), l'épaisseur (e) de la couche de matière isolante (4) placée entre les électrodes (1) et la partie du corps à traiter (A) étant environ comprise entre 0,50 et 3,00 mm, de préférence de l'ordre de 2,00 mm.

2. Dispositif selon la revendication 1, caractérisé en ce que l'élément conducteur (2) est directement posé sur les électrodes (1).

3. Dispositif selon l'une quelconque des revendications 1 et 2, caractérisé en ce qu'il comporte des sangles susceptibles de maintenir les électrodes (1) et la matière isolante (4) dans la position souhaitée en cours de traitement.

## Claims

1. Device for the application of currents at a frequency of between 50 and 300 kHz approximately, without handling, for medical and aesthetic treatment and kinesitherapy, comprising several electrodes (1) which consist of glass tubes placed side by side and in which there is a vacuum of greater or lesser extent, these electrodes (1), whose number and dimensions correspond to the part of the body (A) which it is desired to treat, being connected via a flexible conductor element (2) integral with a socket (3) designed to receive the conductor cable, and the assembly thus obtained being embedded in a flexible insulating material (4) such as a silicone capable of curving inwards to adapt to the part of the body to be treated (A), the thickness (e) of the layer of insulating material (4) placed between the electrodes (1) and the part of the body to be treated (A) being between 0.50 and 3.00 mm approximately, preferably of the order of 2.00 mm.

2. Device according to Claim 1, characterised in that the conductor element (2) is placed directly on the electrodes (1).

3. Device according to either one of Claims 1 and 2, characterised in that it comprises straps capable of holding the electrodes (1) and the insulating material (4) in the desired position during treatment.

## Patentansprüche

1. Einrichtung für die Anwendung von Hochfrequenzströmen zwischen ungefähr 50 und 300 kHz ohne Manipulation bei medizinischen, kosmetischen und heilgymnastischen Behandlungen, welche sich aus mehreren Elektroden (1) zusammensetzt, die aus Seite an Seite angeordneten Glasrohren bestehen, in deren Innerem ein mehr oder weniger starkes Vakuum herrscht, deren Zahl und Abmessungen dem Körperteil (A) entsprechen, welcher behandelt werden soll, und die durch ein weiches Leitungselement (2) verbunden sind, das mit einer Buchse (3) zur Aufnahme des Leitungskabels verbunden ist, wobei die gesamte so erhaltene Anordnung in ein weiches isolierendes Material (4) wie Silikon eingebettet ist, welches zwecks Anschmiegung an den zu behandelnden Körperteil (A) biegsam ist und wobei die Dicke (e) der zwischen den Elektroden (1) und dem zu behandelnden Körperteil (A) gelegenen Schicht des isolierenden Materials (4) etwa zwischen 0,50 und 3,00 mm beträgt, vorzugsweise in der Größenordnung von 2,00 mm.

2. Einrichtung nach Anspruch 1, dadurch gekennzeichnet, daß das Leitungselement (2) direkt auf die Elektroden (1) gelegt ist.

3. Einrichtung nach Anspruch 1 oder 2, dadurch gekennzeichnet, daß sie Grute umfaßt, mittels deren die Elektroden (1) und das isolierende Material (4) bei der Behandlung in der gewünschten Position haltbar sind.

## FIG.1

## FIG. 2

## FIG. 3a

## FIG. 3b

## FIG. 3c